(19) Europäisches Patentamt
European Patent Office
Office européen des brevets

(11) **EP 4 595 873 A1**

(12) **EUROPEAN PATENT APPLICATION**

(43) Date of publication:
06.08.2025   Bulletin 2025/32

(51) International Patent Classification (IPC):
*A61B 5/00* (2006.01)      *A61B 5/02* (2006.01)
*A61B 5/1459* (2006.01)      *G16H 50/20* (2018.01)

(21) Application number: 24154731.4

(22) Date of filing: 30.01.2024

(52) Cooperative Patent Classification (CPC):
**A61B 5/02007; A61B 5/0075; A61B 5/1459;**
**A61B 5/6852; A61B 5/7267; G16H 50/20**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **MUELLER, Manfred**
  **Eindhoven (NL)**
• **LUCASSEN, Gerhardus Wilhelmus**
  **Eindhoven (NL)**
• **BABIC, Drazenko**
  **5656AG Eindhoven (NL)**

(74) Representative: **Philips Intellectual Property &**
**Standards**
**High Tech Campus 52**
**5656 AG Eindhoven (NL)**

(54) **INTRAVASCULAR TISSUE ANALYSIS SYSTEM**

(57)     An intravascular tissue analysis system (100) comprises one or more processors (110) configured to: receive optical data (120) representing an optical property of an intravascular tissue region (130) over one or more wavelength intervals; analyze the optical data (120) to determine i) a blood clot component content (140) of the intravascular tissue region (130), and ii) a lipid content (150) of the intravascular tissue region (130); and output an indication of the blood clot component content and the lipid content, and/or an indication of one or more tissue composition parameters derived therefrom.

FIG. 1

**Description**

TECHNICAL FIELD

The present disclosure relates to performing intravascular tissue analysis. An intravascular tissue analysis system, a computer-implemented method of performing intravascular tissue analysis, and a computer program product, are disclosed.

BACKGROUND

Stroke is a devastating disease, and a leading cause of disability worldwide. Approximately 85% of strokes are caused by ischemia; i.e. the deprivation of blood flow to the brain. The remaining 15% of strokes are caused by a brain haemorrhage; i.e. bleeding in or around the brain.

The main cause of ischemic stroke is thromboembolism. Thromboembolism occurs when a thrombus, also known as a blood clot, develops outside of the brain and is carried via the vasculature to the brain where it forms a blockage. The blockage, also known as an occlusion, leads to cerebral ischemia, and hence to ischemic stroke.

Another possible cause of ischemic stroke is atherosclerosis. Atherosclerosis is a process in which fatty deposits accumulate on the inner walls of the arteries. The fatty deposits are known as atheromas, or plaques. Plaque can grow within a cerebral artery to form a plaque-type occlusion that deprives the brain of blood, similarly leading to cerebral ischemia, and hence to ischemic stroke. This type of atherosclerosis is referred-to as intracranial atherosclerosis. Intracranial atherosclerosis is rare in Western populations but more prevalent in Asian, Black and Hispanic populations.

It is also possible for ischemic stroke to have a mixed origin.

The current treatment options for ischemic stroke include thrombolysis, i.e. the injection of a clot-busting medicine to disburse a blood clot-type occlusion; and mechanical thrombectomy, i.e. an interventional procedure in which an occlusion is mechanically removed from the body. Mechanical thrombectomy involves the insertion of a catheter into the vasculature. The catheter includes a device that captures the occlusion. A variety of mechanical thrombectomy devices are available, including aspiration catheters that are used to suck the occlusion from the vasculature, stent retrievers that include an expandable stent which is used to capture and subsequently extract the occlusion from the body, and expandable mesh disks.

In order to evaluate the different treatment options for ischemic stroke, it is necessary to be able to distinguish between different types of intravascular tissue. This is because different types of occlusions require different treatment options. For instance, stenting is typically used to treat plaque-type occlusions, whereas mechanical thrombectomy is typically used to blood clot-type occlusions. It is possible to further optimize the choice of treatment for blood clot-type occlusions based on the type of tissue present in an occlusion. For example an aspiration catheter is typically better-suited to removing red blood cell-poor blood clots, whereas most stent retrievers are typically better-suited to removing red blood cell-rich blood clots.

Conventionally, the various treatment options for ischemic stroke have been evaluated using non-invasive techniques such as projection X-ray imaging, computed tomography "CT" imaging, and magnetic resonance imaging "MRI". However, these techniques have limitations in terms of their ability to distinguish between different types of occlusions.

Consequently, there is a need to distinguish between different types of intravascular tissue, such as occlusions, in order to assist physicians in evaluating treatment options for ischemic stroke.

Aside from the evaluation of treatment options for ischemic stroke, the ability to distinguish between different types of intravascular tissue would be useful in other clinical situations as well. For instance, this distinction would be useful in assessing treatment options for cardio- and also peripheral vascular disease.

SUMMARY

According to one aspect of the present disclosure, an intravascular tissue analysis system 100, is provided. The system includes one or more processors 110 configured to:

> receive S 110 optical data 120 representing an optical property of an intravascular tissue region 130 over one or more wavelength intervals;
> analyze S120 the optical data 120 to determine i) a blood clot component content 140 of the intravascular tissue region 130, and ii) a lipid content 150 of the intravascular tissue region 130; and
> output S130 an indication of the blood clot component content 140 and the lipid content 150, and/or an indication of one or more tissue composition parameters derived therefrom.

The above system provides the blood clot component content of an intravascular tissue region in combination with the lipid content of the intravascular tissue region. This information facilitates a distinction between intravascular tissue regions that include blood clots, and intravascular tissue regions that include lipids. An example of an intravascular tissue region is an occlusion. The inventors have determined that the lipid content of an occlusion is correlated with the presence of plaque. By providing the blood clot component in addition to the lipid content, the system facilitates a distinction between a blood clot-type occlusion and a plaque-type occlusion. This distinction enables a physician to determine a course of treatment for the occlusion. For instance, if the system indicates that the blood clot component content is relatively high, the physician may conclude that the occlusion is a blood clot-type occlusion. The physician may then choose to remove the occlusion using a mechanical thrombectomy device. By contrast, if the system indicates that the lipid content is relatively high, the physician may conclude that the occlusion is a plaque-type occlusion. The physician may then choose to stent the occlusion. The provision of one or more tissue composition parameters derived from the blood clot component content and the lipid content, similarly facilitates a distinction between intravascular tissue regions that include blood clots, and intravascular tissue regions that include lipids. The tissue composition parameter may for instance be a ratio of the blood clot component content to the lipid content, or an indication of the intravascular tissue region comprising a blood clot, or an indication of the intravascular tissue region comprising plaque. Tissue composition parameters such as these similarly facilitate a distinction between a blood clot-type occlusion and a plaque-type occlusion, and consequently they similarly enable a physician to determine a course of treatment for the occlusion.

Further aspects, features, and advantages of the present disclosure will become apparent from the following description of examples, which is made with reference to the accompanying drawings.

BRIEF DESCRIPTION OF THE DRAWINGS

Fig. 1 is a schematic diagram illustrating an example of an intravascular tissue analysis system 100, in accordance with some aspects of the present disclosure.

Fig. 2 is a flowchart illustrating an example of a computer-implemented method of performing intravascular tissue analysis, in accordance with some aspects of the present disclosure.

Fig. 3 is an example of the optical signatures of various chromophores that are typically present in the vasculature, in accordance with some aspects of the present disclosure.

Fig. 4 is a schematic diagram illustrating an example of an outputted indication of a red blood cell content 140 and a lipid content 150 of an intravascular tissue region, in accordance with some aspects of the present disclosure.

Fig. 5 is an example of the optical signatures of various types of blood clot, blood, a vessel wall, and plaque, in accordance with some aspects of the present disclosure.

Fig. 6 is a schematic diagram illustrating an example of an outputted indication of various types of blood clots, in accordance with some aspects of the present disclosure.

Fig. 7 is a schematic diagram illustrating a first example of an optical probe 180 for insertion into the vasculature, in accordance with some aspects of the present disclosure.

Fig. 8 is a schematic diagram illustrating a second example of an optical probe 180 for insertion into the vasculature, in accordance with some aspects of the present disclosure.

Fig. 9 is a schematic diagram illustrating an example of an optical probe 180 disposed within an intravascular tissue region 130 within a blood vessel 260, in accordance with some aspects of the present disclosure.

DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in a computer program product, in a corresponding manner.

In the following description, reference is made to examples of an intravascular tissue analysis system. The system provides an analysis of an intravascular tissue region. In some examples, reference is made to the use of the system to analyze intravascular tissue in the form of an occlusion within an artery in the brain for the purpose of enabling a physician to select a treatment device for removing the occlusion and thereby treat ischemic stroke. However, it is to be appreciated that unless explicitly stated, the use of the intravascular tissue analysis system disclosed herein is not limited to these examples. Thus, the intravascular tissue analysis system may be used in the analysis of intravascular tissue within the anatomy in general, and the intravascular tissue analysis may be used in various clinical applications. For instance, the intravascular tissue analysis system may be used to analyze intravascular tissue within blood vessels such as arteries, or veins, and such blood vessels may be in anatomical regions such as the brain, the neck, the arm, the leg, and so forth. Moreover, the intravascular tissue analysis system may be used in clinical applications such as the analysis of treatment options for stroke, peripheral vascular disease, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD.

It is also noted that some operations that are described as being performed by the one or more processors of the systems disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

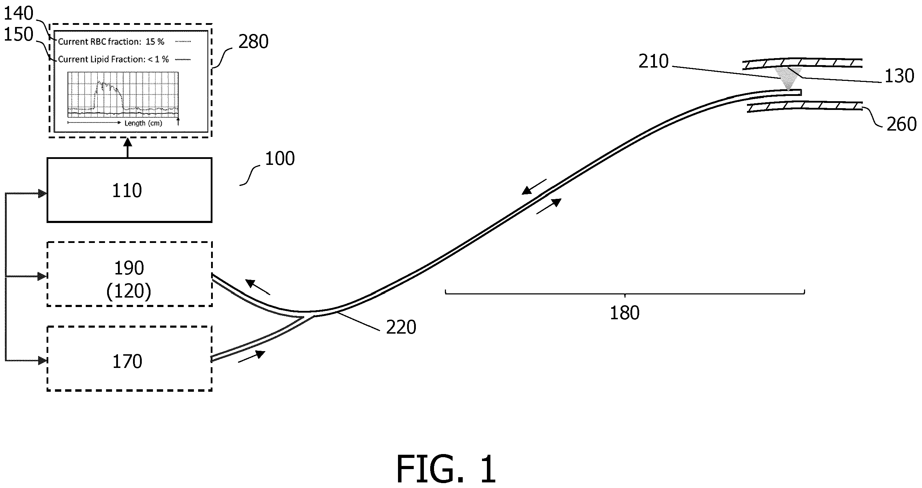

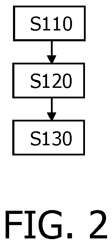

As mentioned above, there is a need to distinguish between different types of intravascular tissue. Fig. 1 is a schematic diagram illustrating an example of an intravascular tissue analysis system 100, in accordance with some aspects of the present disclosure. Fig. 2 is a flowchart illustrating an example of a computer-implemented method of performing intravascular tissue analysis, in accordance with some aspects of the present disclosure. It is noted that operations that are described as being performed by the one or more processors 110 of the intravascular tissue analysis system 100 illustrated in Fig. 1, may also be performed in the method illustrated in Fig. 2. Likewise, operations that are described in relation to the method described with reference to Fig. 2 may also be performed by the one or more processors 110 of the intravascular tissue analysis system 100 illustrated in Fig. 1. With reference to Fig. 1, and Fig. 2, the intravascular tissue analysis system 100, comprises one or more processors 110 configured to:

receive S 110 optical data 120 representing an optical property of an intravascular tissue region 130 over one or more wavelength intervals;

analyze S120 the optical data 120 to determine i) a blood clot component content 140 of the intravascular tissue region 130, and ii) a lipid content 150 of the intravascular tissue region 130; and

output S130 an indication of the blood clot component content 140 and the lipid content 150, and/or an indication of one or more tissue composition parameters derived therefrom.

The above system provides the blood clot component content of an intravascular tissue region in combination with the lipid content of the intravascular tissue region. This information facilitates a distinction between intravascular tissue regions that include blood clots, and intravascular tissue regions that include lipids. An example of an intravascular tissue region is an occlusion. The inventors have determined that the lipid content of an occlusion is correlated with the presence of plaque. By providing the blood clot component in addition to the lipid content, the system facilitates a distinction between a blood clot-type occlusion and a plaque-type occlusion. This distinction enables a physician to determine a course of treatment for the occlusion. For instance, if the system indicates that the blood clot component content is relatively high, the physician may conclude that the occlusion is a blood clot-type occlusion. The physician may then choose to remove the occlusion using a mechanical thrombectomy device. By contrast, if the system indicates that the lipid content is relatively high, the physician may conclude that the occlusion is a plaque-type occlusion. The physician may then choose to stent the occlusion. The provision of one or more tissue composition parameters derived from the blood clot component content and the lipid content, similarly facilitates a distinction between intravascular tissue regions that include blood clots, and intravascular tissue regions that include lipids. The tissue composition parameter may for instance be a ratio of the blood clot component content to the lipid content, or an indication of the intravascular tissue region comprising a blood clot, or an indication of the intravascular tissue region comprising plaque. Tissue composition parameters such as these similarly facilitate a distinction between a blood clot-type occlusion and a plaque-type occlusion, and consequently they similarly enable a physician to determine a course of treatment for the occlusion.

The operations that are performed by the one or more processors of the intravascular tissue analysis system 100 are described in more detail below.

With reference to the method illustrated in Fig. 2, in the operation S110, the one or more processors 110 of the intravascular tissue analysis system 100 receive optical data 120. The optical data 120 represents an optical property of an intravascular tissue region 130 over one or more wavelength intervals.

In general, the optical data 120 that is received in the operation S 110 may represent an optical property such as the reflectance, optical scatter, transmission, absorption, or fluorescence, of the intravascular tissue region 130. In general, the optical data 120 may be generated in response to the irradiation of the intravascular tissue region with optical radiation. In general, the one or more wavelength intervals may occupy a portion of the visible region of the optical spectrum and/or a portion of the near-infrared region of the optical spectrum. In general, the optical data 120 may be generated in real-time, i.e. the optical data 120 may be real-time optical data. Alternatively, the optical data 120 may have been generated historically, i.e. the optical data 120 may be historic optical data.

The optical data 120 that is received in the operation S110 may be received from various sources. For example the optical data 120 may be received from a computer readable storage medium, or from the Internet, or the Cloud, and so forth. In the system 100 illustrated in Fig. 1, the optical data 120 is received from an optical detector 190, and the optical data 120 is generated in real-time. In general, the optical data 120 that is received in the operation S 110 may be received via any form of data communication, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

With continued reference to the method illustrated in Fig. 2, in the operation S 120, the one or more processors 110 of the intravascular tissue analysis system 100, analyze S 120 the optical data 120 to determine i) a blood clot component content 140 of the intravascular tissue region 130, and ii) a lipid content 150 of the intravascular tissue region 130.

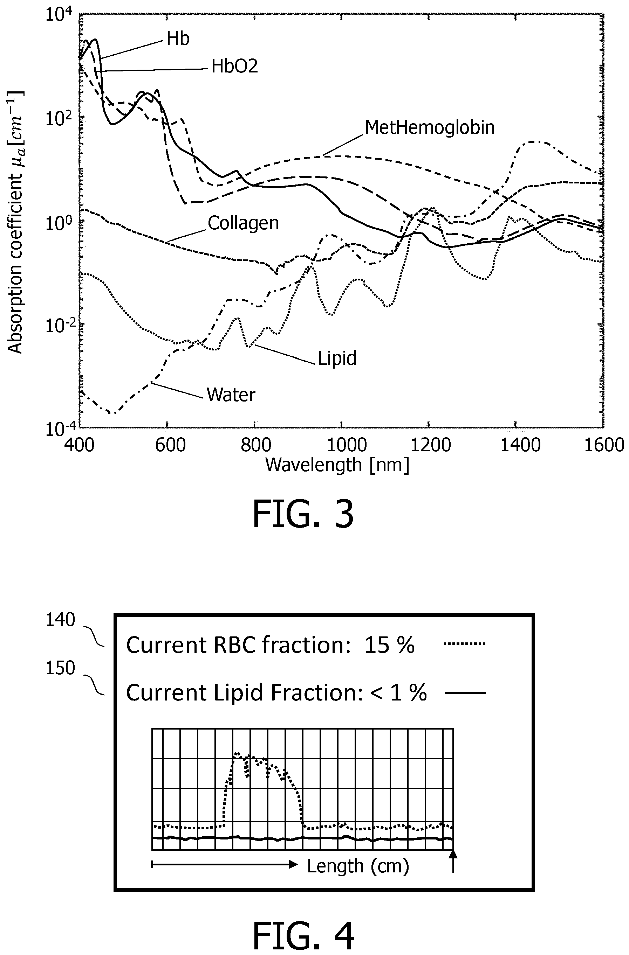

An example of the analysis of optical data in the operation S 120 is now described with reference to Fig. 3, which is an example of the optical signatures of various chromophores that are typically present in the vasculature, in accordance with some aspects of the present disclosure. In general, the blood clot component may include red blood cells and/or fibrin and/or platelets. The blood clot component content 140 may be determined based on a contribution to the optical property caused by red blood cells and/or fibrin and/or platelets, respectively. Similarly, the lipid content 150 may be determined based on a contribution to the optical property caused by lipids. In the example described with reference to Fig. 3, the blood clot component that is determined is the red blood cell component. The content of other blood clot components, such as the fibrin component, and the platelet component, may be determined in a similar manner.

The abscissa in Fig. 3 represents wavelength in nanometers, and the ordinate in of Fig. 3 represents the absorption coefficient of the various chromophores. The chromophores represented in Fig. 3 include water, lipid, collagen, and various haemoglobin chromophores. One of the main constituents of red blood cells is haemoglobin. Haemoglobin is present in the vasculature in various forms, including as deoxygenized haemoglobin, labelled Hb in Fig. 3, and as oxygenized haemoglobin, labelled HbO2 in Fig. 3, and as methaemoglobin.

As may be seen in Fig. 3, within the wavelength interval from approximately 450 nanometers to approximately 800 nanometers, the absorption coefficients of the aforementioned haemoglobin chromophores are relatively high compared to those of other chromophores that are present in the vasculature. In this context, the term "approximately" refers to within $\pm$ 10 nanometers, or within $\pm$ 5 nanometers, of these values. Thus, the red blood cell content 140 of an intravascular region may be determined based on a contribution to the optical absorption coefficient caused by haemoglobin. For example, the red blood cell content of an intravascular region may be determined based on a contribution to the optical absorption coefficient caused by haemoglobin over a wavelength interval within a range extending from approximately 450 nanometers to approximately 800 nanometers. The wavelength interval may be a portion of this range, or it may correspond to the complete range. As described later, the red blood cell content may be used to distinguish between a red blood cell rich environment, such as blood, or a red blood cell-rich occlusion, and a red blood cell poor environment such as a red blood cell-poor occlusion.

As mentioned above, the lipid content of the intravascular tissue region also provides information that facilitates a distinction between different types of intravascular tissue regions. The inventors have determined that the lipid content of an occlusion is correlated with the presence of plaque. Thus, the lipid content may be used to distinguish between a lipid-rich environment, such as a plaque-type occlusion, and a lipid-poor environment, such as a blood clot-type occlusion. As may be seen in Fig. 3, within the wavelength interval from approximately 1100 nanometers to approximately 1300 nanometers, the absorption coefficient of the lipid chromophore has a distinctive peak. In this context, the term "approximately" refers to within ± 10 nanometers, or within ± 5 nanometers, of these values. This peak is distinct from the absorption coefficients of other chromophores in the vasculature such as collagen, haemoglobin, and water. Thus, the lipid content 150 of an intravascular region may be determined based on a contribution to the optical absorption coefficient caused by lipids. For example, the lipid content 150 of an intravascular region may be determined based on a measurement of the optical absorption coefficient over a wavelength interval within a range extending from approximately 1100 nanometers to approximately 1300 nanometers. The wavelength interval may be a portion of this range, or it may correspond to the complete range.

As may be appreciated, instead of measuring the optical absorption (as illustrated in Fig. 3) the measurements of other optical properties of the intravascular tissue region 130 may alternatively be used to determine the red blood cell content and the lipid content of the intravascular tissue region. For instance, measurements of optical properties such as reflectance, or optical scattering, or transmission, or fluorescence, of the intravascular tissue region, are also affected by the chromophores represented in Fig. 3. Consequently measurements of these optical properties may alternatively be used to determine the red blood cell content, and the lipid content, of an intravascular tissue region. The measurements of such optical properties may be obtained using the intravascular tissue analysis system 100 illustrated in Fig. 1, as described in more detail below.

Various techniques may be used to analyze the optical data 120 in the operation S 120 in order to determine the red blood cell content and the lipid content 140 of the intravascular tissue region. In one example, a model fitting technique is used to fit a model to measurements of the optical property. In this example, a model reported in a document by Farrell, T. J. et al., "A diffusion theory model of spatially resolved, steady-state diffuse reflectance for the noninvasive determination of tissue optical properties in vivo", Medical Physics 19(4): p. 879-888 (1992), is fitted to measurements of absorption spectra in order to determine the red blood cell content and the lipid content.

The Farrell model is derived from diffusion theory and describes the optical properties of the tissue via two properties, the absorption coefficient $\mu_a(\lambda)$ and the reduced scattering coefficient $\mu_s'(\lambda)$, both of which are dependent on the wavelength $\lambda$ of the optical radiation. The total absorption coefficient $\mu_a^{total}(\lambda)$ is given by the sum of the absorption coefficients of the various absorbers weighted by their concentrations $c$, i.e.:

$$\mu_a^{total}(\lambda) = c_{Haemoglobin}\mu_a^{Haemoglobin}(\lambda) + c_{Lipid}\mu_a^{Lipid}(\lambda) + c_{Water}\mu_a^{Water}(\lambda)$$
$$+ \cdots. \qquad\qquad\qquad (Equation\ 1)$$

The wavelength-dependent absorption coefficients of Lipids $\mu_a^{Lipid}(\lambda)$, Water $\mu_a^{Water}(\lambda)$, and other absorbers are known from literature. The absorption spectrum of deoxygenated haemoglobin, Hb, is different to that of oxygenated haemoglobin, HbO2. The absorption coefficient due to haemoglobin, $\mu_a^{Haemoglobin}(\lambda)$ is given by:

$$\mu_a^{Haemoglobin}(\lambda) = v(\lambda)\left[StO_2\mu_a^{HbO_2}(\lambda) + (1 - StO_2)\mu_a^{Hb}(\lambda)\right] \qquad (Equation\ 2)$$

where $\mu_a^{Hb}(\lambda)$ and $\mu_a^{HbO_2}(\lambda)$ correspond to absorption coefficients of deoxygenized and oxygenized haemoglobin, and $StO_2$ is the oxygen saturation level of the tissue. The parameter $v(\lambda)$ is used to account for the inhomogeneous distribution of haemoglobin and is known as the "pigment packaging factor" and is given by:

$$v(\lambda) = \frac{1 - \exp\left(-2R\left[StO_2\mu_a^{HbO_2}(\lambda) + (1 - StO_2)\mu_a^{Hb}(\lambda)\right]\right)}{2R\left[StO_2\mu_a^{HbO_2}(\lambda) + (1 - StO_2)\mu_a^{Hb}(\lambda)\right]} \qquad (Equation\ 3)$$

as reported in a document by Verkruysse, W., et al., "Modelling light distributions of homogeneous versus discrete absorbers in light irradiated turbid media", Phys. Med. Biol. 42, 51, and wherein R corresponds to the average radius of a haemoglobin concentration, e.g., a blood vessel. The reduced scattering coefficient is empirically modeled as the sum of Mie and Rayleigh scattering:

$$\mu'_s(\lambda) = \alpha \left[ \rho \left( \frac{\lambda}{\lambda_0} \right)^{-b} + (1 - \rho) \left( \frac{\lambda}{\lambda_0} \right)^{-4} \right] \qquad \text{(Equation 4)}$$

where $\lambda_0 = 800$ nm corresponds to a wavelength normalization value, $\alpha$ is the reduced scattering amplitude at $\lambda_0$ the Mie scattering slope is b, and $\rho$ denotes the Mie-fraction of the reduced scattering in the tissue.

The Farrell model may be fitted to the absorption spectra using a Levenberg-Marquardt non-linear inversion algorithm, for example. The result of the fitting is to determine the values of the concentrations of the haemoglobin and lipid chromophores, i.e. $c_{Haemoglobin}$ and $c_{Lipid}$, and which represent the red blood cell content of the intravascular tissue region, and the lipid content of the intravascular tissue region, respectively.

Instead of the model described in the above example, other models, and also other fitting techniques, may alternatively be used in the operation S 120 to analyze the optical data 120 in order to determine the red blood cell content and the lipid content 140 of the intravascular tissue region. For example, a machine learning algorithm such as a neural network, a decision tree, a support vector machine "SVM", principal component analysis, and so forth, may be trained, and subsequently used, to analyze the optical data 120. The training data used to train the machine learning algorithm may include multiple sets of optical spectra of intravascular tissue regions, and for each set of optical spectra, corresponding ground truth data comprising a ground truth value for the red blood cell content, and a ground truth value for the lipid content. The ground truth data, may be determined via histological analysis of the intravascular tissue regions.

In the above example, the blood clot component content that was determined was that of red blood cells. However, as mentioned above, the content of other blood clot components, such as the fibrin component, and the platelet component, may also be determined in a similar manner based on their optical signatures.

Returning to the method illustrated in Fig. 2, in the operation S 130, the one or more processors 110 of the intravascular tissue analysis system 100, output an indication of the blood clot component content 140 and the lipid content 150, and/or an indication of one or more tissue composition parameters derived therefrom.

The indication of the blood clot component content 140 and the lipid content 150 may be outputted in various forms in the operation S130. For example, the blood clot component content 140 and the lipid content 150 may be outputted in numerical form, or in textual form, or in the form of an icon, or in the form of a graph. The indication of the blood clot component content 140 and the lipid content 150 may be outputted in real-time in the operation S 130. By way of an example, Fig. 4 is a schematic diagram illustrating an example of an outputted indication of a blood clot component content 140 and a lipid content 150 of an intravascular tissue region, in accordance with some aspects of the present disclosure. In the example illustrated in Fig. 4, the blood clot component is the red blood cell component. In the upper portion of Fig. 4, the red blood cell content and the lipid content are outputted as numerical values for an intravascular tissue region 130 that corresponds to a current position of an optical probe in vessel.

In the operation S 130, instead of, or in addition to outputting values for the blood clot component content 140 and the lipid content 150, the processor(s) may output an indication of one or more tissue composition parameters derived from the blood clot component content 140 and the lipid content 150. An example of a tissue composition parameter is a ratio of the blood clot component content 140 to the lipid content 150. Another example of a tissue composition parameter is an indication of the intravascular tissue region comprising a blood clot. An indication of the intravascular tissue region comprising a blood clot may be determined based on the blood clot component content. For instance, a threshold may be applied to the blood clot component content, and if the blood clot component content exceeds the threshold, the intravascular tissue region may be deemed to comprise a blood clot. Another example of a tissue composition parameter is an indication of the intravascular tissue region comprising plaque. An indication of the intravascular tissue region comprising plaque may be determined based on the lipid content. For instance, a threshold may be applied to the lipid content, and if the lipid content exceeds the threshold, the intravascular tissue region may be deemed to comprise plaque.

In one example, the blood clot component content 140 and the lipid content 150 are outputted for each of multiple positions along a vessel. In this example, the received optical data 120 represents the optical property at a plurality of positions along a vessel, and the one or more processors 110 output the blood clot component content 140 and the lipid content 150, and/or the indication of the one or more tissue composition parameters derived therefrom, for each of a plurality of the positions along the vessel.

An example of the outputting of data in accordance with this example is illustrated by the graph in the lower portion of Fig. 4. In this graph, the Fig. 4, the blood clot component is the red blood cell content. In this graph, the abscissa represents the position along the vessel, and the ordinate represents the red blood cell content 140 and the lipid content 150 respectively. The optical data 120 in this example may be obtained during a so-called pullback procedure in which an optical probe 180 that is used to obtain the optical data 120 is translated along the vessel. The position along the vessel may be determined based on a known pullback speed of the optical probe.

The blood clot component content 140 and the lipid content 150 may be outputted to various media in the operation S 130, including to a display such as to the display 280 illustrated in Fig. 1, or to a virtual/ augmented reality display device, or to a printer, or to a computer-readable storage medium, or to the Internet, or to the Cloud, and so forth.

The above-described system provides the blood clot component content of an intravascular tissue region in combination with the lipid content of the intravascular tissue region. This information facilitates a distinction between intravascular tissue regions that include blood clots, and intravascular tissue regions that include lipids. An example of an intravascular tissue region is an occlusion. The inventors have determined that the lipid content of an occlusion is correlated with the presence of plaque. By providing the blood clot component in addition to the lipid content, the system facilitates a distinction between a blood clot-type occlusion and a plaque-type occlusion. This distinction enables a physician to determine a course of treatment for the occlusion. The provision of one or more tissue composition parameters derived from the blood clot component content and the lipid content, similarly facilitates a distinction between intravascular tissue regions that include blood clots, and intravascular tissue regions that include lipids. The tissue composition parameter(s) similarly facilitate a distinction between a blood clot-type occlusion and a plaque-type occlusion, and consequently they similarly enable a physician to determine a course of treatment for the occlusion.

Various further examples of the intravascular tissue analysis system 100 are described below.

In one example, the one or more processors 110 of the intravascular tissue analysis system 100 are configured to analyze the optical data 120 to determine a type of a blood clot 160 in the intravascular tissue region 130; and to output an indication of the type of the blood clot. By outputting an indication of the type of the blood clot, this example provides additional information to a physician that may support the physician in determining a course of treatment for the blood clot.

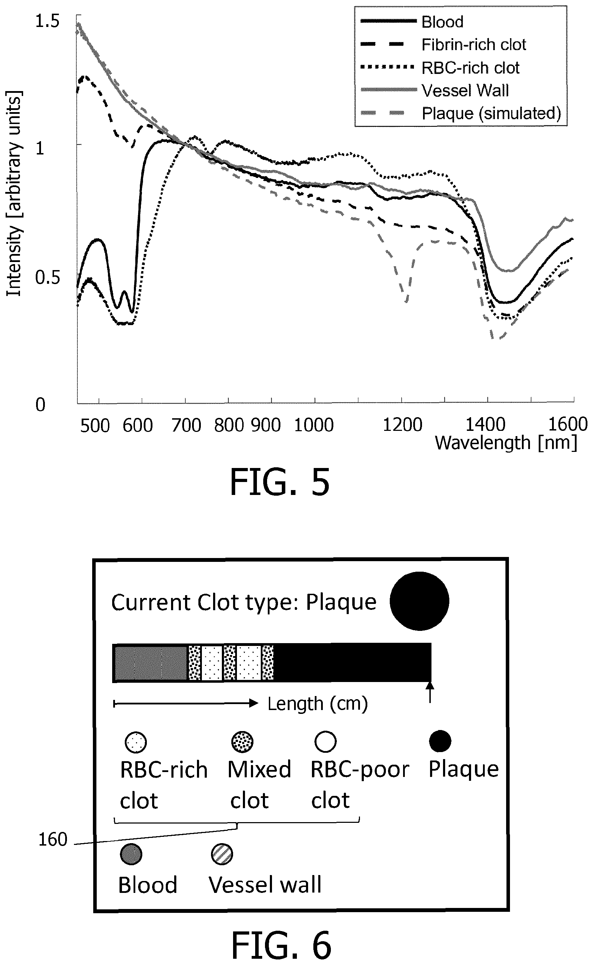

This example is described with reference to Fig. 5, which is an example of the optical signatures of various types of blood clot, blood, a vessel wall, and plaque, in accordance with some aspects of the present disclosure. The abscissa of Fig. 5 represents wavelength in nanometers, and the ordinate axis of Fig. 5 represents the reflectance of the various intravascular media. As may be appreciated from Fig. 5, differences between the spectra of a red blood cell-rich blood clot, a red blood cell-poor blood clot, vessel wall, blood, and plaque, may be used to distinguish these media from one another. For instance, differences between the illustrated spectra enable a determination of a type of a blood clot, the type of the clot being a red blood cell-poor blood clot, a red blood cell-rich blood clot, and a mixed clot. A red blood cell-poor blood clot may alternatively be referred-to as a white clot, or a fibrin-rich blood clot. A red blood cell-rich blood clot may alternatively be referred to as a red clot. A clot that is neither red blood cell-poor, nor red blood cell-rich, may also be referred-to as a mixed clot.

The indication of the type of the blood clot may be outputted in various forms in this example. For example, the type of the blood clot may be outputted in textual form, or in the form of an icon, or in the form of a graph.

By way of an example, Fig. 6 is a schematic diagram illustrating an example of an outputted indication of various types of blood clots, in accordance with some aspects of the present disclosure. In the example illustrated in Fig. 6, the type of the blood clot is outputted in textual form, and also as an icon. The type of the blood clot is also illustrated in the lower portion of Fig. 6 for optical data that is obtained at a plurality of positions along a vessel. Providing the type of the blood clot at multiple positions along a vessel in accordance with this example facilitates an assessment of a length of the blood clot. This information is useful to a physician in determining a course of treatment for the ischemic stroke. For instance it may be used to specify the length of a stent to deploy in the vessel.

In this example the one or more processors 110 may analyze the optical data 120 to determine the type of the blood clot based on a measurement of the optical property over a wavelength interval within a range extending from approximately 450 nanometers to approximately 800 nanometers. The wavelength interval may be a portion of this range, or it may correspond to the complete range. The type of the blood clot that is determined in this example comprises one or more of: a red blood cell-poor blood clot, a red blood cell-rich blood clot, and a mixed clot. The type of the blood clot is determined based on a contribution to the optical property caused by one or more of: a red blood cell-poor blood clot, a red blood cell-rich blood clot, and a mixed clot.

In this example, the contributions to the optical property from the different types of blood clots may be determined by measuring an optical reflectance spectrum of the intravascular tissue region, and then fitting a weighted sum of reference optical signatures for the blood clots, such as the optical signatures of the blood clots illustrated in Fig. 5, to the measured optical reflectance spectrum. The model fitting technique described above may be used to in this operation. The type of the blood clot may then be determined by applying threshold values to the contributions of the different blood clots.

In another example, the one or more processors 110 of the intravascular tissue analysis system 100 are configured to:

analyze the optical data 120 to determine a contribution to the optical property caused by a vessel wall and/or blood 270 in the optical data 120; and
output an indication of the detection of the vessel wall and/or blood, the indication being determined based on the corresponding contribution.

In this example, the contribution to the optical property caused by a vessel wall and/or blood in the optical data 120 may be determined using the model fitting technique described above. These contributions may be determined using reference optical spectra of the vessel wall and/or blood, respectively, such as those illustrated in Fig. 5. As illustrated in Fig. 5, these spectra have characteristic features within the wavelength range extending from approximately 450 nanometers to approximately 800 nanometers. For instance, these spectra have a characteristic absorption edge that may be used to determine their individual contributions to a measured optical reflectance spectrum. The indication of the detection of the vessel wall and/or blood may be outputted in a similar manner to that described above for the blood clots. For instance, as illustrated in Fig. 6, the indication of the detection of the vessel wall and/or blood may be provided in textual form, or in the form of an icon, or in the form of a graph indicating the detection of the vessel wall, or blood, at multiple positions along the vessel.

By providing an indication of the detection of the vessel wall and/or blood in accordance with this example, the system informs a physician of the position of the optical probe in the vasculature. For instance, an indication of blood informs a physician that the optical probe is not currently located within an occlusion. This indication may alert the physician of the need to re-position the optical probe within the occlusion so as to obtain optical data for the occlusion. Similarly, an indication of the detection of the vessel wall may alert the physician of the need to rotate a radially-sensing optical probe in order to instead obtain optical data for the occlusion.

As illustrated in Fig. 1, the optical data 120 that is used to determine the blood clot component content 140 of the intravascular tissue region 130, the lipid content 150 of the intravascular tissue region 130, and likewise the type of a blood clot in the intravascular tissue region 130, may be provided using at least one optical source 170, an optical probe 180 for insertion into the vasculature, and at least one optical detector 190. These elements are now described with reference to Fig. 1 and Fig. 7 - Fig. 9.

In general, the at least one optical source 170 illustrated in Fig. 1 may generate optical radiation over a portion of the visible spectrum and/or over a portion of the near-infrared spectrum. In the example described above, the optical source(s) 170 generate optical radiation over a first wavelength interval that is within a range extending from approximately 450 nanometers to approximately 800 nanometers, and also over a second wavelength interval that is within a range extending from approximately 1100 nanometers to approximately 1300 nanometers. The wavelength intervals may be portions of these ranges, or they may correspond to the complete range. In other examples, the optical source(s) may generate optical radiation over one or more wavelength intervals that are different to this example. In general, the optical source(s) may be provided by lamps, or light emitting diodes "LED"s, or lasers. The at least one optical detector 190 illustrated in Fig. 1 may be provided by various types of detectors, including for example a silicon detector, a gallium arsenide detector, an indium gallium arsenide detector, and so forth.

In some examples, the optical source(s) and the optical detector(s) are configured as a spectrometer. For instance, in one example, a spectrometer may be provided by using a diffractive or refractive optical element to spatially separate the optical radiation into different portions of the wavelength interval(s), and by using one or more optical detectors to detect the intensity the optical radiation over the different portions of the wavelength interval(s). The intensity of the collected optical radiation in each portion of the wavelength interval may be detected using multiple detectors, the spatial positions of which determine the detected portion of the wavelength interval(s), or alternatively a single detector may be used, the position of the single optical detector determining the detected portions of the wavelength interval(s).

In another example, a spectrometer may be provided by using a diffractive, or a refractive, optical element to select from the optical radiation generated by the optical source(s), optical radiation over different portions of the wavelength interval(s). In this example, the spectrometer may be provided by temporally adjusting the position of the diffractive or refractive optical element so as to select optical radiation from different portions of the wavelength interval(s) over time. A corresponding single optical detector may be used to temporally separate the detected optical radiation into the different portions of the wavelength interval(s).

In another example, a spectrometer may be provided by temporally modulating the intensity of different optical sources so as to selectively generate optical radiation over the different portions of the wavelength interval(s). A single optical detector may then be used to temporally separate the collected optical radiation into the different portions of the wavelength intervals.

In another example, a spectrometer may be provided by temporally switching filters that selectively transmit optical radiation over each of the different portions of the of the wavelength interval(s). The filters provide temporally modulated optical radiation for the different portions of the wavelength interval(s). A single optical detector may then be used to temporally separate the collected optical radiation into the different portions of the wavelength interval(s).

In one example, the at least one optical source and the at least one optical detector are configured as a Raman spectrometer. In another example, the at least one optical source and the at least one optical detector are configured to perform diffuse reflectance spectroscopy. In another example, the at least one optical source and the at least one optical detector are configured to perform fluorescence spectroscopy.

Returning to the system 100 illustrated in Fig. 1, the at least one optical source 170 is in optical communication with the optical probe 180, and the optical probe 180 is configured to irradiate the intravascular tissue region 130 with optical radiation 210 generated by the at least one optical source 170, and to collect optical radiation returned by the intravascular tissue region 130 in response to the irradiation. The at least one optical detector 190 is in optical communication with the optical probe 180, and the at least one optical detector 190 is configured measure an intensity of the collected optical radiation over the one or more wavelength intervals to provide the optical data 120.

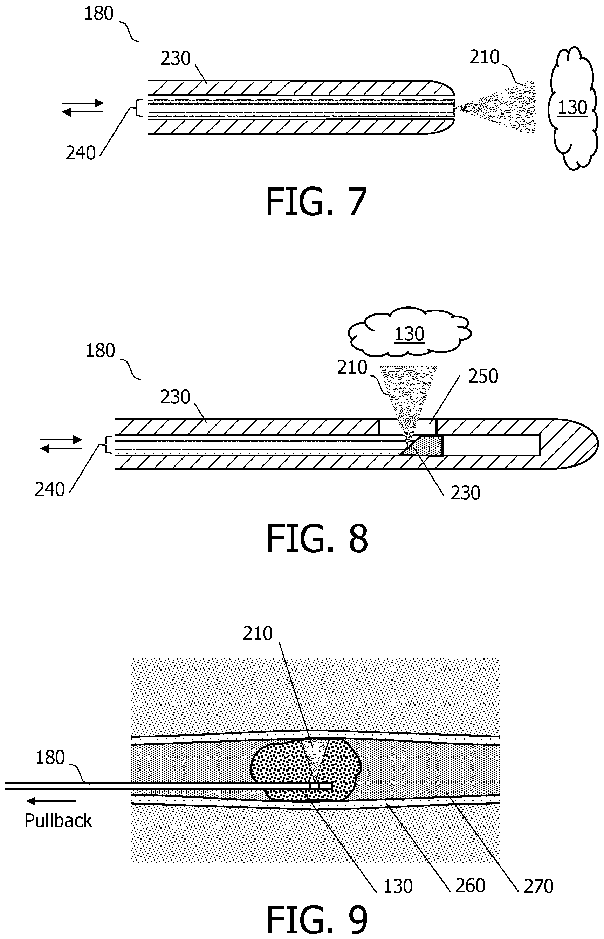

Various types of optical probes may be used with the system illustrated in Fig. 1. In general, these include optical probes that are configured to measure one or more of: optical reflectance, optical scattering, optical transmission, optical absorption, and fluorescence. In general, the optical probe may include one or more optical fibers. For instance, in one example, the optical probe includes a single optical fiber 220, and the single optical fiber is used both to irradiate the intravascular tissue region and to collect optical radiation returned by the intravascular tissue region in response to the irradiation. In another example, the optical probe includes multiple optical fibers, and separate optical fibers are used to irradiate the intravascular tissue region, and to collect optical radiation returned by the intravascular tissue region in response to the irradiation. The use multiple optical fibers offers improved separation between the irradiating, and collected, optical radiation, at the expense of an increase in diameter, and reduced flexibility, of the optical probe. Some examples of optical probes that include a single optical fiber, and which are configured to measure optical reflectance, are described with reference to Fig. 7, and Fig. 8.

Fig. 7 is a schematic diagram illustrating a first example of an optical probe 180 for insertion into the vasculature, in accordance with some aspects of the present disclosure. The optical probe 180 illustrated in Fig. 7 includes an elongate body 230. The elongate body is flexible, and has a smooth outer surface in order to enable its insertion into the vasculature. The optical probe 180 illustrated in Fig. 7 includes a single optical fiber 240. The optical fiber is used both to irradiate an intravascular tissue region 130 and to collect optical radiation returned by the intravascular tissue region in response to the irradiation. The optical fiber 240 is arranged within the elongate body 230. Optical radiation passing along the optical fiber 240 is emitted along a path that is parallel to the longitudinal axis of the optical probe. The optical fiber 240 also collects optical radiation that returns back along the same path as the emitted radiation. This enables the optical probe illustrated in Fig. 7 to analyze an intravascular tissue region 130 that is disposed distally with respect to a distal end of the optical probe. The optical probe illustrated in Fig. 7 may therefore be referred-to as a "forward-sensing" optical probe.

By way of another example, Fig. 8 is a schematic diagram illustrating a second example of an optical probe 180 for insertion into the vasculature, in accordance with some aspects of the present disclosure. As in the Fig. 7 example, the optical probe 180 illustrated in Fig. 8 includes an elongate body 230. The elongate body is flexible, and has a smooth outer surface in order to enable its insertion into the vasculature. As in the Fig. 7 example, the optical probe 180 illustrated in Fig. 8 includes a single optical fiber 240. The optical fiber 240 is used both to irradiate an intravascular tissue region 130 and to collect optical radiation returned by the intravascular tissue region in response to the irradiation. The optical fiber 240 is arranged within the elongate body 230. The optical fiber is arranged within the elongate body 230. In contrast to the Fig. 7 example, the optical probe 180 illustrated in Fig. 8 includes a reflective surface at a beveled end face 230 of the optical fiber 240. The reflective surface may be provided by a mirror, or total internal reflection, for example. The reflective surface redirects optical radiation passing along the optical fiber from a longitudinal direction with respect to the longitudinal axis of the optical probe, and into a radial direction with respect to the longitudinal axis of the optical probe. The emitted optical radiation 210 then passes through an aperture 250 in the elongate tube 230. The optical fiber 240 also collects optical radiation that returns back along the same path as the emitted radiation. This enables the optical probe illustrated in Fig. 8 to analyze an intravascular tissue region 130 that is disposed radially with respect to a distal end of the optical probe. The optical probe illustrated in Fig. 8 may therefore be referred-to as a "radial-sensing" optical probe.

The optical probes illustrated in Fig. 7 and Fig. 8 may be used to analyze intravascular tissue regions such as occlusions in blood vessels. By way of an example, Fig. 9 is a schematic diagram illustrating an example of an optical probe 180 disposed within an intravascular tissue region 130 within a blood vessel 260, in accordance with some aspects of the present disclosure. The optical probe 180 illustrated in Fig. 5 represents an optical probe such as the optical probe 180 illustrated in Fig. 7 or in Fig. 8. The intravascular tissue region 130 illustrated in Fig. 5 may be an occlusion, such as a plaque-type occlusion, or blood clot-type occlusion, for example. As illustrated in Fig. 9, an analysis of the intravascular tissue region 130 may be performed by arranging the distal end of the optical probe 180 from which the optical radiation 210 is emitted, within, abutting, or at least close to, the intravascular tissue region. This analysis may be performed during a so-called pullback procedure in which the optical probe is withdrawn from the vasculature, as illustrated by the arrow labelled Pullback, for example.

Instead of the arrangements illustrated in Fig. 7 and Fig. 8, other types of optical probes may be used with the system illustrated in Fig. 1. These alternative optical probes may have different numbers of optical fibers, and different arrangements of optical fibers. For instance, the distal ends of the optical fiber(s) may be oriented in a different direction to those illustrated in Fig. 7 and Fig. 8 in order to sense a different intravascular tissue region. The distal end(s) of the optical fiber(s) may also be configured to measure another optical property of the intravascular tissue region 130. For instance they may be configured to measure optical scattering, or optical transmission, or optical absorption, or optical fluorescence.

Referring now to the system illustrated in Fig. 1, as mentioned above, the at least one optical source 170 is in optical communication with the optical probe 180, and the at least one optical detector 190 is also in optical communication with the optical probe 180. This optical communication may be provided by one or more optical components such as an optical connector, a beamsplitter, a bifurcated optical fiber, and so forth.

It is noted that the intravascular tissue analysis system 100 may, as illustrated in Fig. 1, also include one or more of: a display, such as the display 280 illustrated in Fig. 1, for displaying the blood clot component content 140, the lipid content 150, the indication of the tissue composition parameter(s) 160, and other outputs generated by the processor(s) 110; and a user input device (not illustrated in Fig. 1) configured to receive user input in relation to the operations performed by the one or more processors 110, such as a keyboard, a mouse, a touchscreen, and so forth.

In another example, a computer-implemented method of performing intravascular tissue analysis, is provided. The method comprises:

> receiving S110 optical data 120 representing an optical property of an intravascular tissue region 130 over one or more wavelength intervals;
> analyzing S120 the optical data 120 to determine i) a blood clot component content 140 of the intravascular tissue region 130, and ii) a lipid content 150 of the intravascular tissue region 130; and
> outputting S130 an indication of the blood clot component content 140 and the lipid content 150, and/or an indication of one or more tissue composition parameters derived therefrom.

In another example, a computer program product is provided. The computer program product comprises instructions which when executed by one or more processors 110, cause the one or more processors to carry out a method of performing intravascular tissue analysis. The method comprises:

> receiving S110 optical data 120 representing an optical property of an intravascular tissue region 130 over one or more wavelength intervals;
> analyzing S120 the optical data 120 to determine i) a blood clot component content 140 of the intravascular tissue region 130, and ii) a lipid content 150 of the intravascular tissue region 130; and
> outputting S130 an indication of the blood clot component content 140 and the lipid content 150, and/or an indication of one or more tissue composition parameters derived therefrom.

An alternative system is now described which determines the presence and/or a type of a blood clot in an intravascular tissue region. This system shares many of the features of the system described above with reference to Fig. 1 and Fig. 2. For instance, it uses the same optical source(s), the same optical detector(s), the same optical probe, and the same optical data as those described above. This alternative system may also include a display and/or a user input device, as described above in relation to Fig. 1. In this alternative system, the optical data may also be analyzed in a similar manner to that described above with reference to Fig. 1 and Fig. 2. For example, the model fitting technique may be used to analyze the optical data. However, this alternative system does not output a blood clot component content 140 and a lipid content 150. Instead, it provides an indication of the presence and/or the type of a blood clot 160, and an indication of the presence and/or a type of a plaque. By providing these parameters directly, the alternative system alleviates the mental burden on the physician of interpreting the meaning of the blood clot component content 140 and lipid content 150.

In this intravascular tissue analysis system, the system comprises one or more processors configured to:

> receive optical data 120 representing an optical property of an intravascular tissue region 130 over one or more wavelength intervals;
> analyze the optical data 120 to determine a) a presence and/or a type of a blood clot 160 in the intravascular tissue region, and b) a presence and/or a type of a plaque in the intravascular tissue region 130; and
> output an indication of a) the presence and/or the type of the blood clot, and b) the presence and/or the type of the plaque.

In this system, the presence of the blood clot is determined based on a contribution to the optical property caused by red blood cells and/or fibrin and/or platelets, and the presence of the plaque is determined based on a contribution to the optical property caused by lipids.

The contribution to the optical property caused by red blood cells and/or fibrin and/or platelets, and lipids may be determined using the model fitting approach described above with reference to Fig. 3. Thus, in one example, the one or more wavelength intervals comprise a first wavelength interval within a range extending from approximately 450 nanometers to approximately 800 nanometers, and a second wavelength interval within a range extending from approximately 1100 nanometers to approximately 1300 nanometers; and the one or more processors are configured to analyze the optical data 120 to determine the presence of the blood clot based on a measurement of the optical property over the first wavelength interval, and to determine the presence of the plaque based on a measurement of the optical property over the second wavelength interval.

The presence of the a blood clot, and the presence of plaque, may be determined by applying threshold values to each contribution to the optical property. For instance, an indication of the presence of a blood clot may be provided if the contribution to the optical property caused by haemoglobin exceeds a specified threshold. Similarly an indication of the presence of plaque may be provided if the contribution to the optical property caused by lipids exceeds a specified threshold.

The type of the blood clot that is determined in this example may be one or more of: a red blood cell-poor blood clot, a red blood cell-rich blood clot, and a mixed clot. The type of the blood clot may be determined in a similar manner to that described above with reference to Fig. 5. Thus, in one example, the one or more wavelength intervals comprise a first wavelength interval within a range extending from approximately 450 nanometers to approximately 800 nanometers, and the type of the blood clot type comprises one or more of: a red blood cell-poor blood clot, a red blood cell-rich blood clot, and a mixed clot. In this example, the one or more processors are configured to analyze the optical data 120 to determine the type of the blood clot based on a measurement of the optical property over the first wavelength interval, and the type of the blood clot is determined based on a contribution to the optical property caused by one or more of: a red blood cell-poor blood clot, a red blood cell-rich blood clot, and a mixed clot.

Similarly, different types of plaque may be identified based on differences in their optical properties. An example of the optical properties, in this example, the reflectance, of one type of plaque are illustrated in Fig. 5, and this has a characteristic absorption band within the wavelength range from approximately 1100 nanometers to approximately 1300 nanometers. Other types of plaque such as calcified plaque, vulnerable plaque, and stable plaque, are expected to have other characteristic features that likewise facilitate their distinction.

In another example, the received optical data 120 represents the optical property at a plurality of positions along a vessel; and the one or more processors are configured to output the indication of a) the presence and/or the type of the blood clot in the intravascular tissue region 130, and b) the presence and/or the type of the plaque in the intravascular tissue region 130, for each of a plurality of the positions along the vessel.

In this example, output may be provided in a similar manner to the graph of the red blood cell fraction versus position along the vessel illustrated in Fig. 4, for example.

In another example, a machine learning algorithm is used to generate the predicted indication of the presence and/or the type of the blood clot, and the predicted indication of the presence and/or the type of the plaque. In this example, the one or more processors are configured to analyze the optical data 120 to determine a) the presence and/or the type of the blood clot in the intravascular tissue region 130, and b) the presence and/or the type of the plaque in the intravascular tissue region 130 by:

> inputting the received optical data 120 into a machine learning algorithm; and
> generating a predicted indication of the presence and/or the type of the blood clot, and a predicted indication of the presence and/or the type of the plaque, in response to the inputting, using the machine learning algorithm; and
> wherein the machine learning algorithm is trained to generate the predicted indication of the presence and/or the type of the blood clot, and the predicted indication of the presence and/or the type of the plaque, from the optical data 120, using training data comprising a plurality of sets of optical training data representing an optical property of an intravascular tissue region 130 over the one or more wavelength intervals; and for each set of optical training data, corresponding ground truth data representing the indication of the presence and/or the type of the blood clot and the indication of the presence and/or the type of the plaque.

In this example, the machine learning algorithm may for example employ a neural network, a decision tree, a support vector machine "SVM", principal component analysis, and so forth.

Examples of neural networks that may be used to generate the predicted indication of the presence and/or the type of the blood clot include recurrent neural networks "RNN"s, encoder/ decoder architectures, variational autoencoders, and so forth. In general, the training of a neural network involves inputting the training data into the neural network, and iteratively adjusting the neural network's parameters until the trained neural network provides an accurate output. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean absolute error (or L1 norm), the mean squared error, the root mean squared error (or L2 norm), the Huber loss, or the (binary) cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem, including gradient descent, Quasi-Newton methods, and so forth. Various algorithms have been developed to implement these methods and their variants, including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers". These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

The training of a neural network is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

In the above example, the neural network may be trained to generate the predicted indication of the presence and/or the type of the blood clot, and the predicted indication of the presence and/or the type of the plaque from the optical data 120, by:

for each of a plurality of sets of the optical training data:

inputting the set of optical training data into the neural network;
generating a predicted indication of the presence and/or the type of the blood clot, and a predicted indication of the presence and/or the type of the plaque, in response to the inputting, using the neural network; and
adjusting parameters of the neural network based on a difference between the predicted indication of the presence and/or the type of the blood clot, and the predicted indication of the presence and/or the type of the plaque, generated by the neural network, and the corresponding indication of the presence and/or the type of the blood clot, and indication of the presence and/or the type of the plaque, from the ground truth data; and
repeating the inputting, the generating, and the adjusting, until a stopping criterion is met.

In this alternative system, the or more processors may also analyze the optical data 120 to determine a contribution to the optical property caused by a vessel wall and/or blood 270 in the optical data 120, and output an indication of the detection of the vessel wall and/or blood, the indication being determined based on the corresponding contribution. This operation may be performed using the model fitting technique described above. A machine learning algorithm may also be used to generated a predicted indication of the detection of the vessel wall and/or blood from the optical data. In this example, the machine learning algorithm may be trained to generate the predicted indication of the detection of the vessel wall and/or blood, from the optical data 120, using training data comprising a plurality of sets of optical training data representing an optical property of an intravascular tissue region 130 over the one or more wavelength intervals; and for each set of optical training data, corresponding ground truth data representing the indication of the detection of the vessel wall and/or blood. The optical training data, and the corresponding ground truth data in this example may be provided from intravascular measurements of the optical property using an optical probe that is known to be in contact with a vessel wall and/or disposed in blood.

In another example, a computer-implemented method of performing intravascular tissue analysis, is provided. The method comprises:

receiving S 110 optical data 120 representing an optical property of an intravascular tissue region 130 over one or more wavelength intervals;
analyzing S 120 the optical data 120 to determine a) a presence and/or a type of a blood clot 160 in the intravascular tissue region, and b) a presence and/or a type of a plaque in the intravascular tissue region 130; and

outputting an indication of a) the presence and/or the type of the blood clot, and b) the presence and/or the type of the plaque.

In another example, a computer program product is provided. The computer program product comprises instructions which when executed by one or more processors 110, cause the one or more processors to carry out a method of performing intravascular tissue analysis. The method comprises:

receiving S 110 optical data 120 representing an optical property of an intravascular tissue region 130 over one or more wavelength intervals;

analyzing S 120 the optical data 120 to determine a) a presence and/or a type of a blood clot 160 in the intravascular tissue region, and b) a presence and/or a type of a plaque in the intravascular tissue region 130; and

outputting an indication of a) the presence and/or the type of the blood clot, and b) the presence and/or the type of the plaque.

The above examples are to be understood as illustrative of the present disclosure, and not restrictive. Further examples are also contemplated. For instance, the examples described in relation to a system, may also be provided by the corresponding computer-implemented method, or by the corresponding computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

**Claims**

1.  An intravascular tissue analysis system (100), the system comprising one or more processors (110) configured to:

receive (S 110) optical data (120) representing an optical property of an intravascular tissue region (130) over one or more wavelength intervals;

analyze (S120) the optical data (120) to determine i) a blood clot component content (140) of the intravascular tissue region (130), and ii) a lipid content (150) of the intravascular tissue region (130); and

output (S130) an indication of the blood clot component content (140) and the lipid content (150), and/or an indication of one or more tissue composition parameters derived therefrom.

2.  The intravascular tissue analysis system according to claim 1, wherein the blood clot component comprises red blood cells and/or fibrin and/or platelets, wherein the blood clot component content (140) is determined based on a contribution to the optical property caused by red blood cells and/or fibrin and/or platelets, respectively, and wherein the lipid content (150) is determined based on a contribution to the optical property caused by lipids.

3.  The intravascular tissue analysis system according to claim 1 or claim 2, wherein the one or more wavelength intervals comprise a first wavelength interval within a range extending from approximately 450 nanometers to approximately 800 nanometers, and a second wavelength interval within a range extending from approximately 1100 nanometers to approximately 1300 nanometers; and

wherein the one or more processors (110) are configured to analyze the optical data (120) to determine i) the blood clot component content (140) based on a measurement of the optical property over the first wavelength interval, and to determine ii) the lipid content (150), based on a measurement of the optical property over the second wavelength interval.

4.  The intravascular tissue analysis system according to any previous claim, wherein the one or more tissue composition parameters comprise one or more of: an indication of the intravascular tissue region (130) comprising a blood clot, and an indication of the intravascular tissue region (130) comprising plaque.

5.  The intravascular tissue analysis system according to any previous claim, wherein the one or more processors (110) are further configured to:

analyze the optical data (120) to determine iii) a type of a blood clot (160) in the intravascular tissue region (130); and

output an indication of the type of the blood clot.

6. The intravascular tissue analysis system according to claim 5, wherein the one or more wavelength intervals comprise a first wavelength interval within a range extending from approximately 450 nanometers to approximately 800 nanometers, and wherein the type of the blood clot comprises one or more of: a red blood cell-poor blood clot, a red blood cell-rich blood clot, and a mixed clot; and

wherein the one or more processors (110) are configured to analyze the optical data (120) to determine iii) the type of the blood clot based on a measurement of the optical property over the first wavelength interval, and wherein the type of the blood clot is determined based on a contribution to the optical property caused by one or more of: a red blood cell-poor blood clot, a red blood cell-rich blood clot, and a mixed clot.

7. The intravascular tissue analysis system according to any previous claim, wherein the received optical data (120) represents the optical property at a plurality of positions along a vessel; and

wherein the one or more processors (110) are configured to output the blood clot component content (140) and the lipid content (150), and/or the indication of the one or more tissue composition parameters derived therefrom, for each of a plurality of the positions along the vessel.

8. An intravascular tissue analysis system, the system comprising one or more processors configured to:

receive optical data (120) representing an optical property of an intravascular tissue region (130) over one or more wavelength intervals;

analyze the optical data (120) to determine a) a presence and/or a type of a blood clot (160) in the intravascular tissue region, and b) a presence and/or a type of a plaque in the intravascular tissue region (130); and

output an indication of a) the presence and/or the type of the blood clot, and b) the presence and/or the type of the plaque.

9. The intravascular tissue analysis system according to claim 8, wherein the one or more processors are configured to analyze the optical data (120) to determine a) the presence and/or the type of the blood clot in the intravascular tissue region (130), and b) the presence and/or the type of the plaque in the intravascular tissue region (130) by:

inputting the received optical data (120) into a machine learning algorithm; and

generating a predicted indication of the presence and/or the type of the blood clot, and a predicted indication of the presence and/or the type of the plaque, in response to the inputting, using the machine learning algorithm; and wherein the machine learning algorithm is trained to generate the predicted indication of the presence and/or the type of the blood clot, and the predicted indication of the presence and/or the type of the plaque, from the optical data (120), using training data comprising a plurality of sets of optical training data representing an optical property of an intravascular tissue region (130) over the one or more wavelength intervals; and for each set of optical training data, corresponding ground truth data representing the indication of the presence and/or the type of the blood clot and the indication of the presence and/or the type of the plaque.

10. The intravascular tissue analysis system according to claim 9, wherein the machine learning algorithm comprises a neural network, and wherein the neural network is trained to generate the predicted indication of the presence and/or the type of the blood clot, and the predicted indication of the presence and/or the type of the plaque from the optical data (120), by:

for each of a plurality of sets of the optical training data:

inputting the set of optical training data into the neural network;

generating a predicted indication of the presence and/or the type of the blood clot, and a predicted indication of the presence and/or the type of the plaque, in response to the inputting, using the neural network; and

adjusting parameters of the neural network based on a difference between the predicted indication of the presence and/or the type of the blood clot, and the predicted indication of the presence and/or the type of the plaque, generated by the neural network, and the corresponding indication of the presence and/or the type of the blood clot, and indication of the presence and/or the type of the plaque, from the ground truth data; and

repeating the inputting, the generating, and the adjusting, until a stopping criterion is met.

11. The intravascular tissue analysis system according to any one of claims 8 - 10, wherein the type of the blood clot comprises one or more of: a red blood cell-poor blood clot, a red blood cell-rich blood clot, and a mixed clot.

12. The intravascular tissue analysis system according to any previous claim, wherein the one or more processors (110) are further configured to:

analyze the optical data (120) to determine a contribution to the optical property caused by a vessel wall and/or blood (270) in the optical data (120); and
output an indication of the detection of the vessel wall and/or blood, the indication being determined based on the corresponding contribution.

13. The intravascular tissue analysis system according to any previous claim, further comprising:

at least one optical source (170);
an optical probe (180) for insertion into the vasculature; and
at least one optical detector (190);
wherein the at least one optical source (170) is in optical communication with the optical probe (180), and the optical probe (180) is configured to irradiate the intravascular tissue region (130) with optical radiation (210) generated by the at least one optical source (170), and to collect optical radiation returned by the intravascular tissue region (130) in response to the irradiation;
wherein the at least one optical detector (190) is in optical communication with the optical probe (180), and the at least one optical detector (190) is configured measure an intensity of the collected optical radiation over the one or more wavelength intervals to provide the optical data (120).

14. A computer-implemented method of performing intravascular tissue analysis, the method comprising:

receiving (S 110) optical data (120) representing an optical property of an intravascular tissue region (130) over one or more wavelength intervals;
analyzing (S120) the optical data (120) to determine i) a blood clot component content (140) of the intravascular tissue region (130), and ii) a lipid content (150) of the intravascular tissue region (130); and
outputting (S130) an indication of the blood clot component content (140) and the lipid content (150), and/or an indication of one or more tissue composition parameters derived therefrom.

15. A computer program product comprising instructions which when executed by one or more processors (110), cause the one or more processors to carry out the method according to claim 14.

FIG. 1

S110

↓

S120

↓

S130

FIG. 2

FIG. 3

FIG. 4

FIG. 5

FIG. 6

FIG. 7

FIG. 8

Pullback

FIG. 9

Europäisches
Patentamt

European
Patent Office

Office européen
des brevets

# EUROPEAN SEARCH REPORT

**Application Number**

EP 24 15 4731

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/101391 A1 (OKADA KAZUNORI [JP] ET AL) 26 April 2012 (2012-04-26) * paragraphs [0019] - [0020], [0042], [0046], [0051] - [0053], [0068] - [0069]; figure 1 * | 1-15 | INV. A61B5/00 A61B5/02 A61B5/1459 G16H50/20 |
| X | JOSE J. RICO-JIMENEZ: "Automatic classification of atherosclerotic plaques imaged with intravascular OCT", BIOMEDICAL OPTICS EXPRESS, vol. 7, no. 10, 15 September 2016 (2016-09-15), page 4069, XP093164350, United States ISSN: 2156-7085, DOI: 10.1364/BOE.7.004069 * abstract, "2 method", "3 results", "4 discussion" * | 1-15 | |
| X | US 2004/111016 A1 (CASSCELLS S WARD [US] ET AL) 10 June 2004 (2004-06-10) * paragraphs [0033] - [0038], [0040], [0044], [0074] - [0100], [0107] - [0108], [0115], [0117], [0164]; claim 1 * | 1-15 | |

TECHNICAL FIELDS SEARCHED (IPC)

A61B
G16H

The present search report has been drawn up for all claims

| Place of search | Date of completion of the search | Examiner |
|---|---|---|
| The Hague | 24 June 2024 | Papanikolaou, V |

EPO FORM 1503 03.82 (P04C01)

## ANNEX TO THE EUROPEAN SEARCH REPORT
## ON EUROPEAN PATENT APPLICATION NO.

EP 24 15 4731

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2024

| Patent document cited in search report | Publication date | Patent family member(s) | | Publication date |
|---|---|---|---|---|
| US 2012101391 A1 | 26-04-2012 | CN | 102421375 A | 18-04-2012 |
| | | EP | 2430980 A1 | 21-03-2012 |
| | | JP | 5647604 B2 | 07-01-2015 |
| | | JP | WO2010131697 A1 | 01-11-2012 |
| | | RU | 2011150502 A | 20-06-2013 |
| | | US | 2012101391 A1 | 26-04-2012 |
| | | WO | 2010131697 A1 | 18-11-2010 |
| US 2004111016 A1 | 10-06-2004 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FARRELL, T. J. et al.** A diffusion theory model of spatially resolved, steady-state diffuse reflectance for the noninvasive determination of tissue optical properties in vivo. *Medical Physics*, 1992, vol. 19 (4), 879-888

- **VERKRUYSSE, W. et al.** Modelling light distributions of homogeneous versus discrete absorbers in light irradiated turbid media. *Phys. Med. Biol.*, vol. 42, 51